# EUROPÄISCHE PATENTSCHRIFT

(11) **EP 0 947 829 B1**
(45) Veröffentlichungstag und Bekanntmachung des Hinweises auf die Patenterteilung: **03.12.2003**
(21) Anmeldenummer: 99106562.4
(22) Anmeldetag: 30.03.1999
(51) Int. Cl.: G01N 27/414, G01N 27/00

(54) **Gassensor zur Detektion von Kohlendioxid durch Messung der Austrittsarbeit von Karbonaten oder Phosphaten**
CO2-sensor based on measurement of work function of carbonates or phosphates
Capteur de CO2 basé sur la mesure du travail d'extraction des carbonates ou phosphates

(30) Priorität: 02.04.1998 DE 19814856
(43) Veröffentlichungstag der Anmeldung: 06.10.1999
(73) Patentinhaber: SIEMENS AKTIENGESELLSCHAFT, 80333 München (DE)
(72) Erfinder: Ostrick, Bernhard, 81541 München (DE); Feischer, Maximilian, Dr., 85635 Höhenkirchen (DE); Meixner, Hans, 85540 Haar (DE)

(56) Entgegenhaltungen:
- DE-A- 4 444 607
- US-A- 4 694 834
- FOGT E. J., UNTEREKER D. F., NORENBERG M. S., MEYERHOFF M.: "RESPONSE OF ION-SELECTIVE FIELD EFFECT TRANSISTORS TO CARBON DIOXIDE AND ORGANIC ACIDS" ANALYTICAL CHEMISTRY, Bd. 57, Nr. 9, August 1985 (1985-08), Seiten 1995-1998, XP002110304
- W. GÖPEL ET AL. (EDITORS): "SENSORS - A COMPREHENSIVE SURVEY, VOL.2, CHEMICAL AND BIOCHEMICAL SENSORS, PART 1" 1991 , VCH PUBLISHERS INC. , NEW YORK XP002102599 SEITEN 467-528, I. LUNDSTRÖM: "FIELD EFFECT CHEMICAL SENSORS" * Seite 473, letzter Absatz *
- QIU F ET AL: "Static characteristic of planar-type CO2 sensor based on NASICON and with an inner-heater" SENSORS AND ACTUATORS B, Bd. 45, Nr. 3, 15. Dezember 1997 (1997-12-15), Seite 233-238 XP004119168 ISSN: 0925-4005
- OSTRICK B ET AL: "Adsorbed water as key to room temperature gas sensitive reactions in work function type sensors: the carbonate-carbon dioxide system" EUROSENSORS XII. PROCEEDINGS OF THE 12TH EUROPEAN CONFERENCE ON SOLID-STATE TRANDUCERS AND THE 9TH UK CONFERENCE ON SENSORS AND THEIR APPLICATIONS, PROCEEDINGS OF EUROSENSORS CONFERENCE, SOUTHAMPTON, UK, 13-16 SEPT. 1998, Seiten 213-216 vol.1, XP002110305 1998, Bristol, UK, IOP Publishing, UKISBN: 0-7503-0536-3

## Beschreibung

Die Erfindung betrifft einen Sensor zur Detektion von Kohlendioxid in Gasgemischen, insbesondere in Luft.

Die Notwendigkeit der Kohlendioxiddetektion ist in vielen Bereichen gegeben. So wird z.B. eine Raumluftüberwachung in Innenräumen zur Feststellung der Raumluftqualität bzw. zur Lüftungs- und Klimaanlagensteuerung vorgenommen. Ein Grenzwert für Kohlendioxid ist beispielsweise 1000 ppm. Darüber hinaus tritt Kohlendioxid gasförmig bei der Lebensmittellagerung und in Gewächshäusern auf, wo es der Luft zugesetzt wird und seine Konzentration überwacht werden muß.

Allgemein werden Sensoren für Kohlendioxid zum einen zur Steuerung der Konzentration in diesen Anwendungen eingesetzt und zum anderen werden auch Sensoren in persönlichen tragbaren Warngeräten erwünscht, wobei Personen mit den Warngeräten in den entsprechenden Anwendungsbereichen arbeiten. Weiterhin könnten solche Warngeräte in Bereichen eingesetzt werden, wo Menschen mit dem Gas *CO*₂ in höheren Konzentrationen, beispielsweise im Prozentbereich, in Berührung kommen. Hier sind beispielsweise Silos oder Weinkeller zu nennen, in denen Lebensgefahr besteht, falls keine Kohlendioxidüberwachung durchgeführt wird.

Bisher bekannte Kohlendioxidsensoren werden beispielsweise durch elektrochemische Zellen dargestellt. Hier ist die Reaktion von Nasicon (*NaO*₂) mit *CO*₂ zu nennen. Zum anderen existieren optische Systeme, die durch selektive Adsorption im nahen Infrarotbereich durch eine Bande im Kohlendioxidspektrum zur Detektion verwendet werden. Nachteile beider Systeme sind zum einen der hohe Preis und zum anderen die Größe des Sensoraufbaues, wodurch vor allem die Anwendung als persönliche Warngeräte behindert wird.

Miniaturisierte Gassensoren auf der Basis von resistiven Messungen an halbleitenden Metalloxiden weisen zum einen das Problem der hohen elektrischen Leistungsaufnahme zur Beheizung von Sensoren auf und zum anderen wird Kohlendioxid durch die bekannten und allgemein verwendeten Materialien für die gassensitive Schicht eines solchen Sensors, wie beispielsweise Zinndioxid, Galliumoxid, Strontium-Titanat ..., nur schlecht detektiert. Darüber hinaus besteht im allgemeinen eine große Querempfindlichkeit gegen Kohlenwasserstoffe. Dies beruht darauf, daß Kohlendioxid an halbleitenden Metalloxiden im Gegensatz zu reduzierenden Gasen, wie Kohlenwasserstoffen oder Kohlenmonoxid nur eine geringe Änderung der Leitfähigkeit dieser Materialien durch Elektroneninjektion bei Adsorbtion des Gases erzeugt. Daher ist auch durch die Entwicklung anderer Metalloxide nicht mit einer zweckmäßigen Weiterentwicklung bestehender Systeme zu rechnen.

Weiterhin bekannt sind Gassensoren nach dem Austritts arbeitsprinzip wie kelvin sonde, SGFET oder CCFET (z.B. DE 4444607).

Das Ziel der Erfindung liegt in der Bereitstellung eines Kohlendioxidsensors in miniaturisierter Form mit verbesserten Meßeigenschaften.

Die Lösung dieser Aufgabe geschieht durch die Merkmale des Anspruchs 1.

Vorteilhafte Ausgestaltungen können den Unteransprüchen entnommen werden.

Der Erfindung liegt die Erkenntnis zugrunde, daß in Verbindung mit der Messung der Austrittsarbeit zur Gasdetektion als gassensitive Schicht ein Karbonat oder ein Phosphat einzusetzen ist. Bisher wurden Karbonate, insbesondere Natriumkarbonat und Bariumkarbonat, bereits als Komponenten in elektrochemischen Zellen zur Detektion von *CO*₂ verwendet z.B. Fabin Qiu et al. Sensors aud Actuators B 45 (1997) 233 - 238. Karbonate zeigen keinerlei Leitfähigkeit, so daß sie nicht erfolgreich als resistive gassensitive Materialien einsetzbar sind. Als isolierende Materialien bieten sie jedoch über die Änderung der Austrittsarbeit an der Oberfläche bei Gasadsorbtion eine gute Meßgröße. Gleiches gilt für Phosphate. Somit sind die nichtleitenden Karbonate und Phosphate, in vorteilhafter Weise für die Detektion von Gassensoren einsetzbar, wobei ein derartiger Sensor mit einer gassensitiven Schicht aus einem Karbonat oder aus einem Phosphat miniaturisierbar ist, ohne elektrische Heizung funktioniert und einfach herstellbar ist.

Im Rahmen der Erfindung werden Karbonate oder Phosphate, z.B. als Teil der Gate-Isolierung (Gatebeschichtung) in gassensitiven Feldeffekttransistoren eingesetzt. Hier ist insbesondere ein hybrider-suspended-Gate-Feldeffekttransistor (HSGFET) zu nennen. In solchen gassensitiven Feldeffekttransistoren sind bisher lediglich Metalle, Metalloxide oder organische Verbindungen als gassensitive Schichten untersucht worden. Diese zeigten jedoch keine signifikante Reaktion auf Kohlendioxid. Insbesondere reagieren die Metalloxide weder bei der Auslesemethode durch Leitfähigkeitsänderungen ( resistive Messungen ) noch durch Änderungen der Austrittsarbeit auf *CO*₂. Karbonate oder Phosphate reagieren sehr wohl mit Änderungen der Austrittsarbeit bei Änderung der CO₂-Konzentration.

Karbonate, deren *CO*₂-Komponenten an der Oberfläche im Gleichgewicht mit dem gasförmigen Kohlendioxid stehen, können besonders vorteilhaft für einen *CO*₂-Sensor eingesetzt werden. Diese Betrachtung kann analog zu dem Gleichgewicht zwischen Gittersauerstoff von Metalloxiden und Luftsauerstoff angestellt werden.

Als Anordnung, die ein Karbonat oder ein Phosphat als gassensitive Schicht enthält, kann eine Kelvinsonde eingesetzt werden. Eine nach der Kelvinmethode funktionierende Sonde ist in der bisherigen Form wesentlich zu groß und zu kostenaufwendig. Ein nach der Erfindung aufgebauter gassensitiver Feldeffekttransistor stellt eine vorteilhafte Möglichkeit zur Messung der Austrittsarbeit dar. Dabei liefert die Änderung der Austrittsarbeit einen zusätzlichen Beitrag zur Gate-Spannung durch den sich der Source-Drain-Strom ändert. Insbesondere die Verwendung eines HSGFET führt zu einem einfachen Meßsystem, da eine derartige gassensitive Schicht leicht darstellbar ist.

Im folgenden werden anhand von schematischen Figuren Ausführungsbeispiele beschrieben.
Figur 1 zeigt den Querschnitt durch einen gassensitiven Feldeffekttransistor,
Figur 2 zeigt einen Ausschnitt eines Meßverlaufes zur Änderung der Austrittsarbeit bei der Detektion von *CO*₂.

Aufbauend auf der Meßmethode, Messung der Austrittsarbeit bei oberflächlicher Gasadsorbtion, ist eine gassensitive Schicht aus einem Karbonat oder aus einem Phosphat hergestellt. Für die Karbonate sind Natriumcarbonat und Bariumcarbonat zu bevorzugen. Für die Phosphate ist Apatit oder Hydroxylapatit mit metallischen Kationen, wie Strontium, Calzium oder Barium vorteilhaft anzuwenden. Darüber hinaus können weitere Carbonate eingesetzt werden. Insgesamt sind Karbonate und Phosphate ideale Materialien zur Darstellung einer gassensitiven Schicht eines beschriebenen Sensors.

Wird beispielsweise eine Kelvinsonde mit einer gassensitiven Schicht aus einem keramischen Pressling aus Bariumkarbonat mit einer Dicke von 1mm erzeugt, so ist ein gewünschter gassensitiver Effekt bei Zimmertemperatur meßbar. Bei einem Wert der maximalen Arbeitsplatzkonzentration (MAK) von Kohlendioxid von 0,5 % ist ein Meßeffekt von bis zu 0,07 mV zu verzeichnen. Insbesondere bewirken Kohlenwasserstoffe in diesem Temperaturbereich keine Querempfindlichkeit.

Die im Vordergrund stehenden Einsatzfälle für eine gassensitive Schicht aus einem Karbonat oder einem Phosphat sind zum einen ein Feldeffekttransistor und zum anderen die Kelvinsonde. Bei einem Feldeffekttransistor ist die entsprechende gas-sensitive Schicht ein Bestandteil der Gate-Isolierung. Bei der Kelvin-Sonde ist die gassensitive Schicht eine Kondensatorelektrode darin.

Bei einer Karbonatschicht beruht die meßbare Sensitivität unter Anwesenheit von Feuchte auf einem Karbonat-Hydroxid-Gleichgewicht an der Oberfläche. Als Schichten für einen Kohlendioxidsensor sind Karbonat-Dünnschichten denkbar. Die gassensitiven Schichten sind in der Regel auf Trägern aufgebracht, so daß sich beispielsweise Oberflächenkarbonat-Schichten in den beschriebenen Sensoren befinden.

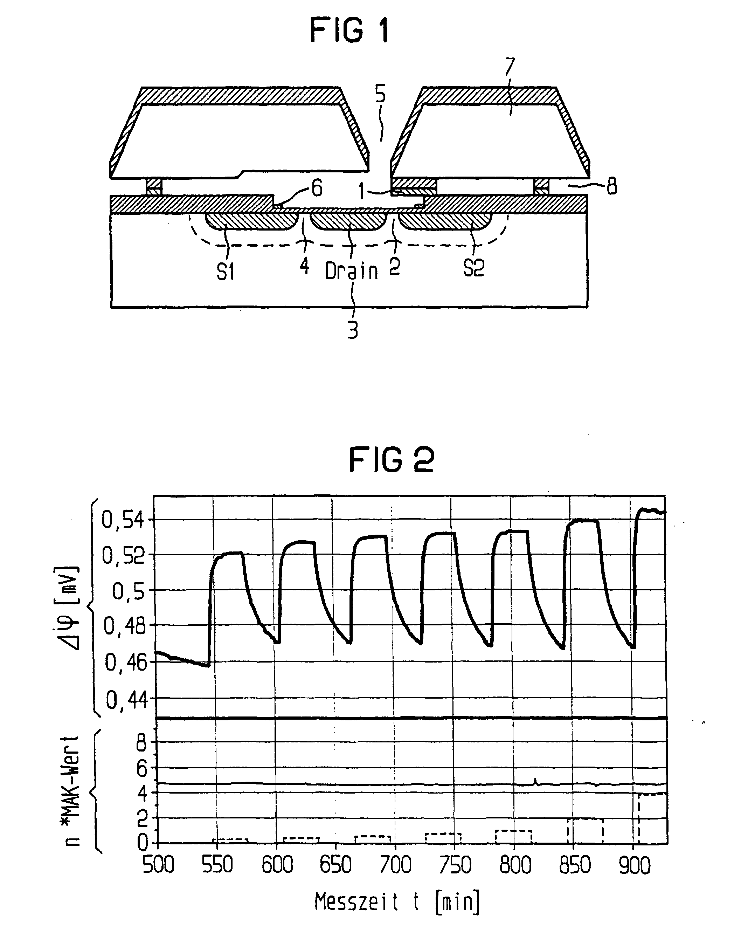

In der Figur 1 wird ein gassensitiver hybrider Feldeffekttransistor mit suspended Gate dargestellt. Dieser besteht aus dem eigenlichen Feldeffekttransistor und einem Referenztransistor. Gekennzeichnet sind ein gemeinsamer Drainbereich 3, Source S1, S2, Rahmen 8, ein Guardring 6 und ein Gaszugang 5. Dabei liegt Source S2 der gassensitiven Schicht 1 gegenüber, wobei Source S1 den Referenz FET darstellt. Der gesamte Aufbau ist auf einer Siliziumbasis verwirklicht. Das hybride Siliziumgate 7 ist in Zusammenhang mit dem Gaszugang 5 derart ausgebildet, daß ein Gasstrom zur gassensitiven Schicht 1 gelangen kann, die in dem Zwischenraum zwischen dem hybriden Siliziumgate 7 und dem Grundkörper der Drain 3 und Source S1, S2 enthält, angeordnet ist. Fertigungstechnisch besteht das Problem die gassensitive Schicht 1 an dieser Stelle darzustellen.

Die Figur 2 zeigt einen Ausschnitt eines Meßverlaufes zur Änderung der Austritsarbeit von Karbonatpreßlingen mit der Kelvin-Methode bei Kohlendioxidbeaufschlagung. Über der Meßzeit t sind die Gaskonzentration von *CO*₂ sowie das Meßsignal Δϕ aufgetragen.

## Patentansprüche

1. Gassensor zur Detektion von Kohlendioxid, bei dem die Austrittsarbeit einer gassensitiven Schicht gemessen wird, wobei die gassensitive Schicht aus einer festen Phase eines Karbonates oder eines Phosphates besteht.

2. Gassensor nach Anspruch 1, wobei die gassensitive Schicht aus Karbonaten von Lithium, Natrium, Magnesium, Calcium, Strontium, Barium, Mangan, Cobalt, Nickel oder Kupfer besteht.

3. Gassensor nach Anspruch 1, wobei die gassensitive Schicht im Falle des Phosphates aus Apatit oder Hydroxylapatit mit Strontium oder Calcium oder Barium oder einer Kombination daraus als metallische Kationen besteht.

4. Gassensor nach einem der vorhergehenden Ansprüche, wobei die gassensitive Schicht in einem Feldeffekttransistor (FET) als Bestandteil der Gate-Isolierung dargestellt ist.

5. Gassensor nach Anspruch 4, wobei der Feldeffekttransistor ein Suspended Gate-FET (SGFET), ein Hybrider-SGFET (HSGFET) oder ein Capacitively-Coupled-FET (CCFET) ist.

6. Gassensor nach einem der Ansprüche 1-3, wobei die gassensitive Schicht als Kondensatorelektrode in einer Kelvinsonde dargestellt ist.

## Claims

1. Gas sensor for the detection of carbon dioxide, in which the work function of a gas-sensitive layer is measured, the gas-sensitive layer consisting of a solid phase of a carbonate or a phosphate.

2. Gas sensor according to Claim 1, in which the gas-sensitive layer consists of carbonates of lithium, sodium, magnesium, calcium, strontium, barium, manganese, cobalt, nickel or copper.

3. Gas sensor according to Claim 1, in which in the case of phosphate the gas-sensitive layer consists of apatite or hydroxylapatite with strontium or calcium or barium or a combination thereof as metallic cations.

4. Gas sensor according to one of the preceding claims, in which the gas-sensitive layer is formed in a field-effect transistor (FET) as part of the gate insulation.

5. Gas sensor according to Claim 4, in which the field-effect transistor is a suspended gate FET (SGFET), a hybrid SGFET (HSGFET) or a capacitively coupled FET (CCFET).

6. Gas sensor according to one of Claims 1-3, in which the gas-sensitive layer is formed as capacitor electrode in a Kelvin probe.

## Revendications

1. Capteur de gaz pour la détection de CO₂, dans lequel le travail d'extraction d'une couche sensible aux gaz est mesuré, la couche sensible aux gaz étant constituée d'une phase solide d'un carbonate ou d'un phosphate.

2. Capteur de gaz selon la revendication 1, la couche sensible aux gaz étant constituée de carbonates de lithium, de sodium, de magnésium, de calcium, de strontium, de baryum, de manganèse, de cobalt, de nickel ou de cuivre.

3. Capteur de gaz selon la revendication 1, la couche sensible aux gaz étant constituée dans le cas du phosphate d'apatite ou d'hydroxylapatite avec du strontiun ou du calcium ou du baryum ou d'une combinaison de ceux-ci comme cations métalliques.

4. Capteur de gaz selon l'une des revendications précédentes, la couche sensible aux gaz étant préparée dans un transistor à effet de champ (FET) comme composant de l'isolation de grille.

5. Capteur de gaz selon la revendication 4, le transistor à effet de champ étant un FET à grille suspendue (SGFET), un SGFET hybride (HSGFET) ou un FET couplé de manière capacitive (CCFET).

6. Capteur de gaz selon l'une des revendications 1 à 3, la couche sensible aux gaz étant préparée comme électrode à condenseur dans une sonde de Kelvin.
